# EUROPEAN PATENT SPECIFICATION

(11) **EP 3 592 857 B1**
(45) Date of publication and mention of the grant of the patent: **29.06.2022**
(21) Application number: 18708434.8
(22) Date of filing: 07.03.2018
(51) Int. Cl.: C12P 7/04

(54) **BIOTECHNOLOGICAL METHOD FOR PRODUCING ALLYL ALCOHOL**
BIOTECHNOLOGISCHES VERFAHREN ZUR HERSTELLUNG VON ALLYLALKOHOL
MÉTHODE BIOTECHNOLOGIQUE POUR LA PRODUCTION D'ALCOOL ALLYLIQUE

(30) Priority: 08.03.2017 EP 17159875
(43) Date of publication of application: 15.01.2020
(73) Proprietor: Evonik Operations GmbH, 45128 Essen (DE)
(72) Inventor: HAAS, Thomas, 48161 Münster (DE); DEMLER, Martin, 46286 Dorsten (DE)
(74) Representative: Evonik Patent Association
(86) International application number: PCT/EP2018/055646
(87) International publication number: WO 2018/162578

(56) References cited:
- US-A1- 2012 252 082
- DEMIRER G N ET AL: "Anaerobic biotransformation of four 3-carbon compounds (acrolein, acrylic acid, allyl alcohol and N-propanol) in UASB reactors", WATER RESEARCH, ELSEVIER, AMSTERDAM, NL, vol. 32, no. 3, March 1998 (1998-03), pages 747-759, XP004107547, ISSN: 0043-1354, DOI: 10.1016/S0043-1354(97)00369-2
- ROGERS ET AL: "Clostridium acetobutylicum Mutants That Produce Butyraldehyde and Altered Quantities of Solvents.", APPLIED AND ENVIRONMENTAL MICROBIOLOGY, vol. 53, no. 12, December 1987 (1987-12), pages 2761-2766, XP055021139, ISSN: 0099-2240
- HARIS NALAKATH ABUBACKAR ET AL: "Ethanol and Acetic Acid Production from Carbon Monoxide in a Clostridium Strain in Batch and Continuous Gas-Fed Bioreactors", INTERNATIONAL JOURNAL OF ENVIRONMENTAL RESEARCH AND PUBLIC HEALTH, vol. 12, no. 1, 20 January 2015 (2015-01-20), pages 1029-1043, XP055401210, DOI: 10.3390/ijerph120101029
- COTTER J L ET AL: "Influence of process parameters on growth of Clostridium ljungdahlii and Clostridium autoethanogenum on synthesis gas", ENZYME AND MICROBIAL TECHNOLOGY, STONEHAM, MA, US, vol. 44, no. 5, 6 May 2009 (2009-05-06), pages 281-288, XP026004172, ISSN: 0141-0229, DOI: 10.1016/J.ENZMICTEC.2008.11.002 [retrieved on 2008-11-17]

## Description

### FIELD OF THE INVENTION

The present invention relates to a biotechnological method for production of allyl alcohol. In particular, the method relates to a biotechnological production of allyl alcohol from acrylic acid in the presence of an acetogenic bacteria.

### BACKGROUND OF THE INVENTION

Allyl alcohol is used as a raw material for the production of glycerol, medicines, perfumes, cosmetics, pesticides and the like. In particular, allyl alcohol may be used for the production of other organic chemicals such as 1,4-butanediol, epichlorohydrin, n-propyl alcohol, isobutyl alcohol and the like.

Since allyl alcohol is known to be toxic in nature, and is even considered to be more toxic than other typical small alcohols, allyl alcohol has always been produced using chemical means. Some conventional processes for the manufacture of allyl alcohol usually involves at least one of the following methods: (a) the alkaline hydrolysis of allyl chloride, (b) the oxidation of propylene to acrolein, followed by the reaction of the acrolein with a secondary alcohol to form allyl alcohol and a ketone, (c) isomerization of propylene oxide using a lithium phosphate catalyst, or (d) hydrolysis of allyl acetate. In order to effectively carry out the latter method of hydrolysis of allyl acetate, an azeotropic distillation step has even been designed. However, the conversion rate of allyl acetate is still restricted by the complexity of the components involved in the process such as allyl acetate, allyl alcohol, water and esters thereof which will be present together in the distillation column. As can be seen, none of these methods offer a direct one-step synthesis of allyl alcohol.

There is therefore a need in the art for a process for producing allyl alcohol using a simplified, cheap and efficient process.

### DESCRIPTION OF THE INVENTION

The present invention attempts to solve the problems above by providing a biotechnological means of producing an allyl alcohol. In particular, the method according to any aspect of the present invention enables the production of allyl alcohol from acrylic acid in the presence of carbon monoxide and at least one acetogenic bacteria. In one example, hydrogen may also be present in the method.

According to one aspect of the present invention, there is provided a method of producing allyl alcohol from acrylic acid, the method comprising contacting at least one acetogenic bacteria with (i) an aqueous medium comprising acrylic acid and/or an acrylate and (ii) a gas mixture comprising carbon monoxide, wherein the acetogenic bacteria is selected from the group consisting of *Alkalibaculum bacchi* DSM 22112, *Clostridium aceticum* DSM 1496, *Clostridium autoethanogenum* DSM 10061, DSM 19630 and DSM 23693, *Clostridium carboxidivorans* DSM 15243, *Clostridium coskatii* ATCC no. PTA-10522, *Clostridium drakei* ATCC BA-623, *Clostridium formicoaceticum* DSM 92, *Clostridium glycolicum* DSM 1288, *Clostridium ljungdahlii* DSM 13528, *Clostridium ljungdahlii* C-01 ATCC 55988, *Clostridium ljungdahlii ERI-2* ATCC 55380, *Clostridium ljungdahlii O-52*ATCC 55989, *Clostridium mayombei* DSM 6539, *Clostridium methoxybenzovorans* DSM 12182, *Clostridium ragsdalei* DSM 15248, *Clostridium scatologenes* DSM 757, and *Clostridium species* ATCC 29797.

Allyl alcohol is also known as prop-2-en-1-ol and has a structural formula of CH₂=CHCH₂OH. Surprisingly, producing allyl alcohols, which are known to be more toxic than other related alcohols, using the method according to any aspect of the present invention does not kill the acetogenic bacteria immediately. Further, the method according to any aspect of the present invention provides a one step process for producing allyl alcohol without the use of expensive and complicated equipment. The conversation of acrylic acid to allyl alcohol also results in no loss of carbon atoms as a 3 carbon molecule is converted to another 3 carbon molecule. The method thus provides an efficient means for producing allyl alcohol. The method according to any aspect of the present invention also provides for a method of producing allyl alcohol from a new substrate-acrylic acid and/or salts thereof.

The term "acetogenic bacteria" as used herein refers to a microorganism which is able to perform the Wood-Ljungdahl pathway and thus is able to convert CO and/or CO₂ with or without hydrogen to acetate. These microorganisms include microorganisms which in their wild-type form do not have a Wood-Ljungdahl pathway, but have acquired this trait as a result of genetic modification. Such microorganisms include but are not limited to *E*. *coli* cells. These microorganisms may be also known as carboxydotrophic bacteria. Currently, 21 different genera of the acetogenic bacteria are known in the art (Drake, H.L., Acetogenic prokaryotes M. Dworkin, S. Falkow, E. Rosenberg, K.-H. Schleifer, E. Stackebrandt (Eds.) (3rd ed.), The Prokaryotes, vol. 2, Springer-Verl (2006)) ), and these may also include some *clostridia* (Drake, HL and Küsel, K: Acetogenic clostridia. In: Dürre, P. (ed.), Handbook on Clostridia, CRC-Press, 719-746 (2005)). These bacteria are able to use carbon dioxide or carbon monoxide as a carbon source with hydrogen as an energy source (Wood, H.G., 1991. Life with CO or CO2 and H2 as a source of carbon and energy FASEB J. 5 156-163). Further, alcohols, aldehydes, carboxylic acids as well as numerous hexoses may also be used as a carbon source (Drake, H. L. et al., 2004. Physiology of the thermophilic acetogen Moorella thermoacetica. Research in Microbiology 155: 422-436). The reductive pathway that leads to the formation of acetate is referred to as acetyl-CoA or Wood-Ljungdahl pathway.

In particular, the acetogenic bacteria may be selected from the group consisting of *Alkalibaculum bacchi* DSM 22112, *Clostridium aceticum* DSM 1496, *Clostridium autoethanogenum* DSM 10061, DSM 19630 and DSM 23693, *Clostridium carboxidivorans* DSM 15243, *Clostridium coskatii* ATCC no. PTA-10522, *Clostridium drakei* ATCC BA-623, *Clostridium formicoaceticum* DSM 92, *Clostridium glycolicum* DSM 1288, *Clostridium ljungdahlii* DSM 13528, *Clostridium ljungdahlii C-01* ATCC 55988, *Clostridium ljungdahlii ERI-2* ATCC 55380, *Clostridium ljungdahlii O-52* ATCC 55989, *Clostridium mayombei* DSM 6539, *Clostridium methoxybenzovorans* DSM 12182, *Clostridium ragsdalei* DSM 15248, *Clostridium scatologenes* DSM 757, *Clostridium species* ATCC 29797 *(*Schmidt et al., 1986, Chem. Eng. Commun., Vol. 45, p. 61-73*)*. More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium ljungdahlii.* In particular, strains selected from the group consisting of *Clostridium ljungdahlii* PETC, *Clostridium ljungdahlii* ERI2, *Clostridium ljungdahlii* COL and *Clostridium ljungdahlii* O-52 may be used in the conversion of synthesis gas to hexanoic acid. These strains for example are described in WO 98/00558, WO 00/68407, ATCC 49587, ATCC 55988 and ATCC 55989. Even more in particular, the acetogenic bacteria may be selected from the group consisting of *Clostridium ljungdahlii* DSM 13528, *Clostridium ljungdahlii C-01* ATCC 55988, *Clostridium ljungdahlii ERI-2* ATCC 55380, *Clostridium ljungdahlii O-52* ATCC 55989, *Clostridium autoethanogenum* DSM 10061, *Clostridium autoethanogenum* DSM 19630 and *Clostridium autoethanogenum* DSM 23693.. In one example, the acetogenic cell used according to any aspect of the present invention may be *Clostridium autoethanogenum* DSM 10061.

As used herein, "aqueous medium" refers to any medium where the solvent is water. In particular, the aqueous medium according to any aspect of the present invention may be any suitable medium where cells can grow. A skilled person would be able to easily identify the suitable medium based on the cell used. In particular, the aqueous medium may comprise at least one of the following components MgCl₂ × 6 H₂O, NaCl, CaCl₂ × 2 H₂O, NaH₂PO₄ × 2 H₂O, KCI, NH₄Cl, nitrilotriacetic acid, MgSO₄ x 7 H₂O, MnSO₄ x H₂O, FeSO₄ x 7 H₂O, Fe(SO₄)₂(NH₄)₂ x 6 H₂O, CoCl₂ x 6 H₂O, ZnSO₄ x 7 H₂O, CuCl₂ x 2 H₂O, KAl(SO₄)₂ x 12 H₂O, H₃BO₃, Na₂MoO₄ x 2 H₂O, Na₂SeO₃, NiCl₂ x 6 H₂O, Na₂WO₄ x 6 H₂O, d-biotin, folic acid, pyridoxine-HCI, thiamine-HCI, riboflavin, nicotinic acid, Ca-pantothenate, vitamin B₁₂, p-aminobenzoate, lipoic acid and FeCl₃. More in particular, the aqueous medium may comprise 0.5 g L⁻¹ MgCl₂ × 6 H₂O, 0.21 g L⁻¹ NaCl, 0.125 g L⁻¹ CaCl₂ x 2 H₂O, 2.65 g L⁻¹ NaH₂PO₄x 2 H₂O, 0.5 g L⁻¹ KCI, 2.5 g L⁻¹ NH₄Cl, 15 mg L⁻¹ nitrilotriacetic acid, 30 mg L⁻¹ MgSO₄ x 7 H₂O, 5 mg L⁻¹ MnSO₄ x H₂O, 1 mg L⁻¹ FeSO₄ x 7 H₂O, 8 mg L⁻¹ Fe(SO₄)₂(NH₄)₂ x 6 H₂O, 2 mg L⁻¹ COCl₂ x 6 H₂O, 2 mg L⁻¹ ZnSO₄ x 7 H₂O, 0.2 mg L⁻¹ CuCl₂ x 2 H₂O, 0.2 mg L⁻¹ KAl(SO₄)₂ x 12 H₂O, 3 mg L⁻¹ H₃BO₃, 0.3 µg L⁻¹ Na₂MoO₄ x 2 H₂O, 0.2 mg L⁻¹ Na₂SeO₃, 0.2 mg L⁻¹ NiCl₂ x 6 H₂O, 0.2 m,g L⁻¹ Na₂WO₄ x 6 H₂O, 20 µg L⁻¹ d-biotin, 20 µg L⁻¹ folic acid, 10 µg L⁻¹ pyridoxine-HCI, 50 µg L⁻¹ thiamine-HCI, 50 µg L⁻¹ riboflavin, 50 µg L⁻¹ nicotinic acid, 50 µg L⁻¹ Ca-pantothenate, 50 µg L⁻¹ vitamin B₁₂, 50 µg L⁻¹ p-aminobenzoate, 50 µg L⁻¹ lipoic acid, and 10 mg L⁻¹ FeCl₃. In one example, the aqueous medium may also comprise L-cysteine-hydrochloride. In another example, the aqueous medium may also comprise sodium acrylate. A skilled person would be able to easily vary the components of the aqueous medium for efficient growth of the cells.

The aqueous medium may comprise acrylic acid and/or acrylate. In one example, the aqueous medium comprises acrylic acid only. In this example, the acrylic acid used according to any aspect of the present invention may be crude acrylic acid. Acrylic acid also known as 2-propenoic acid, may be obtainable by at least catalytic gas-phase oxidation of propane, propene and/or acrolein. The acrylic acid may then be separated by fractional condensation. This acrylic acid may be called crude acrylic acid and contain a range of different impurities that may be a result of the gas-phase oxidation process.

In another example, the crude acrylic acid may be purified to separate off the impurities. Purification of crude acrylic acid may be carried out by rectification, crystallization or the like. In particular, the acrylic acid used according to any aspect of the present invention may have purity ≧ 98% by weight, based on the sum of all components present, i.e. it contains at least 98%, based on its weight, of acrylic acid molecules. In particular, pure acrylic acids are therefore in particular those acrylic acids whose purity, based in the same way as above on the sum of all components present, is ≧ 98.5 or ≧ 99 or ≧ 99.5 or ≧ 99.75 or ≧ 99.9% by weight. This may be referred to as "pure acrylic acid". In one example according to any aspect of the present invention, pure acrylic acid may be used as the substrate for producing allyl alcohol.

In yet another example, the source of acrylic acid in the aqueous medium may be an acrylate. In particular, the acrylate may be a salt, ester, or conjugate base of acrylic acid and derivatives of acrylic acid thereof. In particular, the source of acrylate may be any alkali or alkaline earth acrylate. More in particular, the acrylate may be selected from the group consisting of methyl acrylate, ethyl acrylate, pentabromophenyl acrylate, sodium acrylate, potassium acrylate, lithium acrylate, magnesium acrylate, zinc acrylate, calcium acrylate, ammonium acrylate and Zirconium acrylate. Even more in particular, the source of acrylic acid in the aqueous medium according to any aspect of the present invention may be sodium acrylate.

In a further example, the source of acrylic acid in the aqueous medium may be a combination of acrylic acid and at least one acrylate.

The concentration of acrylic acid and/or acrylate in the aqueous medium at any point in time when the method according to any aspect of the present invention is carried out, or during the method according to any aspect of the present invention may be within the range of 50-10000mg/L. In particular, the concentration of the acrylic acid and/or acrylate in the aqueous medium may be 100-5000mg/L or 100-2000mg/L. In one example, the concentration of the acrylic acid and/or acrylate in the aqueous medium may be 20-450mg/L. The concentration of the acrylic acid and/or acrylate in the aqueous medium may be measured using any method known in the art. In particular, the method used may be nuclear magnetic resonance spectrometry. The concentration of acrylic acid and/or acrylate in the aqueous medium may be maintained at 22-430mg/L in the aqueous medium. In particular, the concentration acrylic acid and/or acrylate in the aqueous medium may be maintained at 25-450, 30-450, 40-450, 50-450, 60-450, 70-450, 80-450, 90-450, 100-450, 150-450, 25-400, 30-400, 40-400, 50-400, 60-400, 70-400, 80-400, 90-400, 100-400, 150-400, 25-350, 30-350, 40-350, 50-350, 60-350, 70-350, 80-350, 90-350, 100-350, 150-350, 25-300, 30-300, 40-300, 50-300, 60-300, 70-300, 80-300, 90-300, 100-300, 150-300, 60-200, 70-200, 80-200, 90-200, 100-200, 50-190, 50-180, 50-170, 50-150, 50-100 mg/L and the like. More in particular, the concentration of acrylic acid and/or acrylate in the aqueous medium may be maintained at about 20, 25, 30, 35, 40, 45, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450 mg/L and like.

In one example, the acrylic acid and/or acrylate may be present in the aqueous medium prior to introduction of the acetogenic cells into the medium. In another example, the acrylic acid and/or acrylate may be introduced into the aqueous medium where the acetogenic cells were already present before.

As used herein, the terms "about" and "approximately", as applied to one or more particular cell culture conditions refer to a range of values that are similar to the stated reference value for that culture condition or conditions. In certain examples, the term "about" refers to a range of values that fall within 25, 20, 19, 18, 17, 16, 15, 14, 13, 12, 11, 10, 9, 8, 7, 6, 5, 4, 3, 2, 1 percent or less of the stated reference value for that culture condition or conditions. For example, an amount of an ingredient employed in a mixture when modified by "about" includes the variation and degree of care typically employed in measuring in an experimental condition in production plant or lab. For example, the amount of a component of a product when modified by "about" includes the variation between batches in multiple experiments in the plant or lab and the variation inherent in the analytical method. Whether or not modified by "about," the amounts include equivalents to those amounts. Any quantity stated herein and modified by "about" can also be employed in the present invention as the amount not modified by "about." In particular, the term 'about 100mg/L of acrylic acid and/or acrylate' may refer to the concentration of 70mg/L, 75mg/L, 80mg/L, 85mg/L, 90mg/L, 95mg/L, 100mg/L, 105mg/L, 110mg/L, 115mg/L, 120mg/L, 125mg/L, and the like. In another example, the term 'about 200mg/L of acrylic acid and/or acrylate' may refer to the concentration of 175mg/L, 180mg/L, 185mg/L, 190mg/L, 195mg/L, 200mg/L, 205mg/L, 210mg/L, 215mg/L, 220mg/L, 225mg/L and the like.

The concentration of acrylic acid and/or acrylate in the medium used according to any aspect of the present invention may be maintained to maintain the allyl alcohol production and/or the survival of the cells. The concentration of the acrylic acid and/or acrylate in the aqueous medium used according to any aspect of the present invention may be maintained at the specific concentrations mentioned. In one example, the concentration of acrylic acid and/or acrylate is at least substantially maintained at the concentration mentioned above, for example at least above 0.0g/L and equal to or below about 100mg/L and the like. A skilled person would be capable of maintaining the concentration of acrylic acid and/or acrylate at the desired level by methods known in the art. In particular, the skilled person would regularly measure the concentration of acrylic acid and/or acrylate in the aqueous medium and adjust the concentration of acrylic acid and/or acrylate accordingly by adding a higher or lower concentration of acrylic acid and/or acrylate into the medium. In one example, the acrylic acid and/or acrylate may be added to the aqueous medium in a continuous flow separately from the continuous feed of the aqueous medium. In another example, acrylic acid and/or acrylate may be part of the aqueous medium that is being topped up. In particular, acrylic acid and/or acrylate may be fed to the aqueous medium as part of the nutrient feed or separately. Whichever route is taken to feed acrylic acid and/or acrylate to the aqueous medium, a skilled person would understand the means to maintain the concentration of acrylic acid and/or acrylate in the aqueous medium. In one example, the acrylic acid and/or acrylate concentration in the medium may be maintained by topping up the acrylic acid and/or acrylate every about 2, 4, 5, 6, 8, 10, 12, 14, 15, 16, 18, or 20 hours of fermentation.

In one example, the concentration of acrylic acid and/or acrylate in the aqueous medium is maintained at any of the concentrations used according to any aspect of the present invention for 80% of the reaction period. In another example, the concentration of acrylic acid and/or acrylate is maintained for 50%, 55%, 60%, 65%, 70%, 75%, 85%, 90%, 95% or 100% of the reaction time. In this regard, reaction time' refers to the period during with a process takes place. In particular, reaction time refers to the period of time from when a reaction starts to when the reaction ends and/or is completed, i.e. where the substrate is used up or when the cells are not capable of converting the acrylic acid to allyl alcohol or when there are other limitations that end the reaction process and no more desired product such as allyl alcohol according to the method of the present invention is produced. In one example, the substrate may be acrylic acid and/or acrylate, and the reaction is completed when the acrylic acid and/or acrylate in the fermenter is used up and the reaction stops and no further acrylic acid and/or acrylate is fed into the fermenter. Therefore, the reaction time refers to the period from which the fermentation starts (i.e. when the acrylic acid and/or acrylate first comes into contact with at least one acetogenic bacteria in a fermenter in suitable fermentation conditions) to when the fermentation ends (i.e. when there is no more acrylic acid and/or acrylate in the fermenter and/or when there is another limiting factor in the fermenter that stops the reaction from continuing). In one example, the reaction period may be for 24hr, 48hr, 72hr, 96hr, 108hr, 120hr, 132hr, 144hr, 156hr, 168hr, 180hr, 192hr, 204hr and the like. In another example, the reaction period may be for 165, 170, 175, 185, 190, and the like hours.

An increase in concentration of acrylic acid and/or acrylate in the aqueous medium may lead to an increase in allyl alcohol produced.

Several factors may affect the acrylic acid conversion to allyl alcohol and the selectivity of allyl alcohol production vis-a-vis by-product production according to any aspect of the present invention. These factors include, for example: the contact time of the acetogenic cell to the aqueous medium, the composition of the feed gas mixture, the temperature of the feed gas mixture, the pH of the fermentation medium, the concentration of medium components, the pressure of the feed gas mixture, the ratio of allyl alcohol to acrylic acid and the like.

The gas mixture comprising carbon monoxide (CO) may be provided to the aqueous medium in a continuous gas flow. It was surprisingly found that the presence of CO in the gas mixture allows for the conversion of acrylic acid and/or acrylate in the aqueous medium to allyl alcohol. Without CO, this conversion may not take place.

CO concentration in the gas flow may be present at least at a measurable concentration. In one example, the concentration of CO may be 0.1% by volume of the volume of the total amount of gas in the gas flow. In another example, the concentration of CO may be 2% by volume of the volume of the total amount of gas in the gas flow. In particular, the CO may be present at a concentration range of 0.1 to 60% by volume, 1 to 50% by volume, 1 to 40% by volume, 2 to 99% by volume, at a range of 2 to 95 % by volume, at a range of 2 to 40 % by volume, 5 to 95% by volume, at a range of 5 to 50 % by volume, at a range of 5 to 20 % by volume, 10 to 90% by volume, 15 to 85% by volume, 15 to 50% by volume, 10 to 20% by volume, particularly at a range of 20 to 80% by volume. More in particular, the concentration of CO may be about 5%, 10%, 15%, 20%, 30%, 40%, 50%, 60% or the like by volume. Gas phase concentration of carbon monoxide in the carbon source may be measured using at least a gas chromatograph GC 6890N of Agilent Technologies Inc. with a thermal conductivity detector. In one example, the CO may be part of synthesis gas. All percentages (%) are, unless otherwise specified, volume percent.

According to any aspect of the present invention, a reducing agent, for example hydrogen may be part of the gas mixture. In particular, the hydrogen may be supplied together with the CO. In particular, this hydrogen may be supplied when the CO is supplied and/or used. In one example, the hydrogen gas is part of synthesis gas. In another example, where the hydrogen gas in the synthesis gas is insufficient for the method of the present invention, additional hydrogen gas may be supplied. In particular, the gas mixture comprises 20-90% by volume hydrogen gas. More in particular, the hydrogen may be present at a concentration range of 30 to 85% by volume, at a range of 40 to 90 % by volume, at a range of 40 to 80 % by volume, 50 to 70% by volume, 50 to 85% by volume, 15 to 85% by volume, 15 to 50% by volume, particularly at a range of 20 to 80% by volume. More in particular, the concentration of H₂ may be about 50%, 55%, 60% or the like by volume.

In one example, the gas mixture further comprises carbon dioxide (CO₂). In particular, the CO₂ may be supplied together with the CO. In particular, this CO₂ may be supplied when the CO is supplied and/or used. In one example, the CO₂ is part of synthesis gas. In particular, the gas mixture comprises 2-50% by volume CO₂. More in particular, the CO₂ may be present at a concentration range of 10 to 50% by volume, at a range of 15 to 50% by volume, 20 to 50% by volume, 30 to 50% by volume, 10 to 40% by volume, 10 to 30% by volume, particularly at a range of 15 to 25% by volume. More in particular, the concentration of CO₂ may be about 15%, 25%, 20% or the like by volume.

A skilled person would understand the other conditions necessary to carry out the method according to any aspect of the present invention. In particular, the conditions in the container (e.g. fermenter) may be varied depending on the acetogenic bacteria used. The varying of the conditions to be suitable for the optimal functioning of the microorganisms is within the knowledge of a skilled person.

In one example, the method according to any aspect of the present invention may be carried out in an aqueous medium with a pH between 5 and 8, 5.5 and 7. In particular, the pH of the aqueous medium may be maintained at pH 5 to 6. More in particular, the pH may be about 5.5 throughout the reaction time. A skilled person may use simple method known in the art to maintain the pH in the aqueous medium. For example, manual and/or automatic addition of ammonium hydroxide may be carried out in the aqueous medium to maintain the pH of the medium.

The pressure may be between 1 and 10 bar.

According to any aspect of the present invention, the gas mixture may comprise or may be synthesis gas. Synthesis gas can for example be produced as a by-product of coal gasification. Accordingly, the microorganism according to any aspect of the present invention may be capable of converting a substance which is a waste product into a valuable resource. In another example, synthesis gas may be a by-product of gasification of widely available, low-cost agricultural raw materials for use in the method of the present invention.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂. Syngas may also be a product of electrolysis of CO₂. A skilled person would understand the suitable conditions to carry out electrolysis of CO₂ to produce syngas comprising CO in a desired amount.

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

In particular, the aqueous medium may be fed with the gas mixture comprising CO and H₂ or just CO. More in particular, the gas mixture may be provided to the aqueous medium in a continuous gas flow. Even more in particular, the continuous gas flow comprises synthesis gas. In one example, the gases are part of the same flow/stream. In another example, each gas is a separate flow/stream provided to the aqueous medium. These gases may be divided for example using separate nozzles that open up into the aqueous medium, frits, membranes within the pipe supplying the gas into the aqueous medium and the like.

A skilled person would understand that it may be necessary to monitor the composition and flow rates of the streams at relevant intervals. Control of the composition of the stream can be achieved by varying the proportions of the constituent streams to achieve a target or desirable composition. The composition and flow rate of the blended stream can be monitored by any means known in the art. In one example, the system is adapted to continuously monitor the flow rates and compositions of at least two streams and combine them to produce a single blended substrate stream in a continuous gas flow of optimal composition, and means for passing the optimised substrate stream to the mixed culture according to any aspect of the present invention.

The term "contacting", as used herein, means bringing about direct contact between the acetogenic bacteria according to any aspect of the present invention and the acrylic acid and/or acrylate and/or CO and/or H₂. For example, the cell in the fermentation medium and the CO and/or acrylic acid and/or acrylate may be in different compartments. In particular, the CO may be in a gaseous state and added to the fermentation medium comprising the cells according to any aspect of the present invention.

In another example, the aqueous medium may be continuously exchanged for new aqueous medium. This allows for the fermentation process to last longer as the stable conditions are maintained by constant change in the aqueous medium.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### No conversion of acrylic acid to allyl alcohol during batch autotrophic cultivation of Clostridium autoethanogenum on synthesis gas without carbon monoxide

For a potential conversion of acrylic acid to the corresponding alcohol allyl alcohol the acetogenic bacterium *Clostridium autoethanogenum* DSM 10061 is cultivated on synthesis gas without carbon monoxide. All cultivation steps are carried out under anaerobic conditions in pressure-resistant glass bottles that were closed airtight with butyl rubber stoppers.

For the pre-culture 500 ml of a defined medium is inoculated with 5 mL of a frozen cryo stock of *C*. *autoethanogenum.* The defined medium was composed as follows: 0.5 g L⁻¹ MgCl₂ x 6 H₂O, 0.21 g L⁻¹ NaCl, 0.125 g L⁻¹ CaCl₂ x 2 H₂O, 2.65 g L⁻¹ NaH₂PO₄ × 2 H₂O, 0.5 g L⁻¹ KCI, 2.5 g L⁻¹ NH₄Cl, 15 mg L⁻¹ nitrilotriacetic acid, 30 mg L⁻¹ MgSO₄ x 7 H₂O, 5 mg L⁻¹ MnSO₄ x H₂O, 1 mg L⁻¹ FeSO₄ x 7 H₂O, 8 mg L⁻¹ Fe(SO₄)₂(NH₄)₂ x 6 H₂O, 2 mg L⁻¹ CoCl₂ x 6 H₂O, 2 mg L⁻¹ ZnSO₄ x 7 H₂O, 0.2 mg L⁻¹ CuCl₂ × 2 H₂O, 0.2 mg L⁻¹ KAl(SO₄)₂ × 12 H₂O, 3 mg L⁻¹ H₃BO₃, 0.3 µg L⁻¹ Na₂MoO₄ × 2 H₂O, 0.2 mg L⁻¹ Na₂SeO₃, 0.2 mg L⁻¹ NiCl₂ × 6 H₂O, 0.2 m,g L⁻¹ Na₂WO₄ × 6 H₂O, 20 µg L⁻¹ d-biotin, 20 µg L⁻¹ folic acid, 10 µg L⁻¹ pyridoxine-HCI, 50 µg L⁻¹ thiamine-HCI, 50 µg L⁻¹ riboflavin, 50 µg L⁻¹ nicotinic acid, 50 µg L⁻¹ Ca-pantothenate, 50 µg L⁻¹ vitamin B₁₂, 50 µg L⁻¹ p-aminobenzoate, 50 µg L⁻¹ lipoic acid, 10 mg L⁻¹ FeCl₃, 20 g L⁻¹ N-morpholinoethansulfonic acid (MES) with additional 500 mg L⁻¹ L-cysteine-hydrochloride. The pH at the beginning is set to pH = 6.0. The autotrophic cultivation is carried out in a 1L pressure-resistant glass bottle at 37 °C, an agitation rate of 150 min⁻¹ and a gassing rate of 1 L h⁻¹ with a synthesis gas consisting of 67% H₂ and 33% CO₂ in an open water bath shaker Innova 3100 from New Brunswick. The gas is dispersed into the medium through a microbubble sparger with a pore size of 10 µm, which is mounted in the center of the reactors. The pH in the pre-cultivation is not controlled.

After the pre-cultivation, an adequate amount of cell suspension is centrifuged (10 min, 4200 min⁻¹) in order to reach an OD₆₀₀ₙₘ of 0.5 in the main cultivation. The pellets are resuspended in fresh medium and transferred into the main cultivation vessel. The main experiment is carried out in the same defined medium as described above, but without MES. Acrylic acid is added to medium at a concentration of 150 mg L⁻¹ in the form of sodium acrylic acid prior to inoculation. The pH at the beginning was set to 6.0 and is later held constant at a pH of 5.5 by automatic addition of ammonium hydroxide (2.5 M). The autotrophic cultivation is carried out in a 1L pressure-resistant glass bottle with a liquid volume of 250 mL at 37 °C and an agitation rate of 150 min⁻¹ in an open water bath shaker Innova 3100 from New Brunswick. The synthesis gas consisting of 67% H₂ and 33% CO₂ is dispensed into the headspace of the reactor a gassing rate of 1 L h⁻¹. During the autotrophic cultivations, 5 mL samples are taken to determine OD₆₀₀ₙₘ, pH und product formation. The determination of the product concentrations is performed by semiquantitative 1H-NMR spectroscopy. As an internal quantification standard sodium trimethylsilylpropionate (T(M)SP) is used.

During the autotrophic cultivation, acetate is the main product, ethanol is only marginally produced. Similarly, no conversion of acrylic acid to allyl alcohol could be measured.

### Example 2

### Conversion of acrylic acid to allyl alcohol with resting cells of Clostridium autoethanogenum on synthesis gas with carbon monoxide

For a potential conversion of acrylic acid to allyl alcohol with synthesis gas containing 20% carbon monoxide resting cells of the acetogenic bacterium *Clostridium autoethanogenum* DSM 10061 were used. All cultivation and conversion steps were carried out under anaerobic conditions in pressure-resistant glass bottles that were closed airtight with butyl rubber stoppers.

For the pre-culture 500 ml of a defined medium were inoculated with 5 mL of a frozen cryo stock of *C. autoethanogenum.* The defined medium was composed as follows: 0.5 g L⁻¹ MgCl₂ × 6 H₂O, 0.21 g L⁻¹ NaCl, 0.125 g L⁻¹ CaCl₂ x 2 H₂O, 2.65 g L⁻¹ NaH₂PO₄ × 2 H₂O, 0.5 g L⁻¹ KCI, 2.5 g L⁻¹ NH₄Cl, 15 mg L⁻¹ nitrilotriacetic acid, 30 mg L⁻¹ MgSO₄ x 7 H₂O, 5 mg L⁻¹ MnSO₄ x H₂O, 1 mg L⁻¹ FeSO₄ x 7 H₂O, 8 mg L⁻¹ Fe(SO₄)₂(NH₄)₂ x 6 H₂O, 2 mg L⁻¹ CoCl₂ x 6 H₂O, 2 mg L⁻¹ ZnSO₄ x 7 H₂O, 0.2 mg L⁻¹ CuCl₂ x 2 H₂O, 0.2 mg L⁻¹ KAI(SO₄)₂ x 12 H₂O, 3 mg L⁻¹ H₃BO₃, 0.3 µg L⁻¹ Na₂MoO₄ x 2 H₂O, 0.2 mg L⁻¹ Na₂SeO₃, 0.2 mg L⁻¹ NiCl₂ x 6 H₂O, 0.2 m,g L⁻¹ Na₂WO₄ x 6 H₂O, 20 µg L⁻¹ d-biotin, 20 µg L⁻¹ folic acid, 10 µg L⁻¹ pyridoxine-HCI, 50 µg L⁻¹ thiamine-HCI, 50 µg L⁻¹ riboflavin, 50 µg L⁻¹ nicotinic acid, 50 µg L⁻¹ Ca-pantothenate, 50 µg L⁻¹ vitamin B₁₂, 50 µg L⁻¹ p-aminobenzoate, 50 µg L⁻¹ lipoic acid, 10 mg L⁻¹ FeCl₃, 20 g L⁻¹ N-morpholinoethansulfonic acid (MES) with additional 500 mg L⁻¹ L-cysteine-hydrochloride. The pH at the beginning was set to pH = 6.0. The autotrophic cultivation was carried out in a 1L pressure-resistant glass bottle at 37 °C, an agitation rate of 150 min⁻¹ and a gassing rate of 1 L h⁻¹ with a synthesis gas consisting of 60% H₂, 20% CO and 20% CO₂ in an open water bath shaker Innova 3100 from New Brunswick. The gas was dispersed into the medium through a microbubble sparger with a pore size of 10 µm, which was mounted in the center of the reactors. The pH in the pre-cultivation was not controlled. After the pre-cultivation, an adequate amount of cell suspension was centrifuged (10 min, 4200 min⁻¹) in order to reach an OD₆₀₀ₙₘ of 0.8-0.9 in the main cultivation. The pellets were resuspended in fresh medium and transferred into the main cultivation vessels. The main experiments were carried out in the same defined medium as described above, but without MES. In the control experiment (exp 2.1) (Table 1), propionic acid was added at a concentration of 1200 mg L⁻¹ in the form of sodium propionate prior to inoculation. In experiment 2.2 acrylic acid was added to the medium at a concentrations of 400 mg L⁻¹ in the form of sodium acrylate prior to inoculation. The pH at the beginning was set to 6.0 and later was held constant at a pH of 5.0 by automatic addition of ammonium hydroxide (2.5 M). The autotrophic cultivations were carried out in 1L pressure-resistant glass bottles with a liquid volume of 250 mL at 37 °C and an agitation rate of 150 min⁻¹ in an open water bath shaker Innova 3100 from New Brunswick. The synthesis gas consisting of 60% H₂, 20% CO and 20% CO₂ was dispensed into the headspace of the reactor with a gassing rate of 1 L h⁻¹. During the autotrophic cultivations, 5 mL samples were taken to determine OD₆₀₀ₙₘ, pH und product formation. The determination of the product concentrations was performed by semiquantitative 1H-NMR spectroscopy. As an internal quantification standard sodium trimethylsilylpropionate (T(M)SP) was used. The results are shown in Table 1.

In the control cultivation, cells began to grow until an OD₆₀₀ of 1.49 after 112 hours of cultivation. Acetate and ethanol were produced to concentrations of up to 10000 mg L⁻¹ and 3300 mg L⁻¹ respectively.

A conversion of propionate to propanol was measured simultaneously to the conversion of acetate to ethanol.

Surprisingly, a conversion of acrylic acid to allyl alcohol up to a maximum concentration of 200 mg L⁻¹ was also measured simultaneously to the conversion of acetate to ethanol in experiment 2.2. (Table 1) But a dramatic negative influence on growth and product formation was measured in this experiment. No growth could be measured during the cultivation and OD₆₀₀ declined instead until the end of the experiment.

**Table 1. Results of experiments 2.1 and 2.2**

| **experiment 2.1 (control)** | | | NMR analytics | | | |
|---|---|---|---|---|---|---|
| time, h | pH | OD 600 nm | acetate, mg/L | ethanol, mg/L | propionate, mg/L | propanol, mg/L |
| 0,0 | 5,89 | 0,92 | 1 | 18 | 1250 | n.d. |
| 15,4 | 5,16 | 1,13 | 1200 | 700 | 330 | 430 |
| 23,0 | 4,98 | 1,20 | 1500 | 1200 | 290 | 490 |
| 39,5 | 4,98 | 1,30 | 3100 | 2100 | 240 | 490 |
| 47,3 | 4,99 | 1,33 | 3600 | 2250 | 230 | 500 |
| 111,7 | 4,99 | 1,49 | 10300 | 3300 | 180 | 440 |

| **experiment 2.1** | | | | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,97 | 0,81 | 0 | 7 | 430 | n.d. |
| 15,4 | 5,55 | 0,75 | 670 | 670 | 120 | 180 |
| 23,0 | 5,63 | 0,74 | 660 | 1100 | 84 | 200 |
| 39,5 | 5,73 | 0,56 | 450 | 1200 | 60 | 170 |
| 47,3 | 5,57 | 0,39 | 450 | 1250 | 63 | 200 |
| 111,7 | 5,40 | 0,31 | 430 | 1000 | 55 | 120 |

### Example 3

### Conversion of acrylic acid to allyl alcohol during batch autotrophic cultivation of Clostridium autoethanogenum on synthesis gas with carbon monoxide

For a potential conversion of acrylic acid to allyl alcohol the acetogenic bacterium *Clostridium autoethanogenum* DSM 10061 was cultivated on synthesis gas with 20% carbon monoxide. All cultivation steps were carried out under anaerobic conditions in pressure-resistant glass bottles that were closed airtight with butyl rubber stoppers.

For the pre-culture 500 ml of a defined medium were inoculated with 5 mL of a frozen cryo stock of *C. autoethanogenum.* The defined medium was composed as follows: 0.5 g L⁻¹ MgCl₂ × 6 H₂O, 0.21 g L⁻¹ NaCl, 0.125 g L⁻¹ CaCl₂ x 2 H₂O, 2.65 g L⁻¹ NaH₂PO₄ × 2 H₂O, 0.5 g L⁻¹ KCI, 2.5 g L⁻¹ NH₄Cl, 15 mg L⁻¹ nitrilotriacetic acid, 30 mg L⁻¹ MgSO₄ x 7 H₂O, 5 mg L⁻¹ MnSO₄ x H₂O, 1 mg L⁻¹ FeSO₄ x 7 H₂O, 8 mg L⁻¹ Fe(SO₄)₂(NH₄)₂ x 6 H₂O, 2 mg L⁻¹ CoCl₂ x 6 H₂O, 2 mg L⁻¹ ZnSO₄ x 7 H₂O, 0.2 mg L⁻¹ CuCl₂ x 2 H₂O, 0.2 mg L⁻¹ KAI(SO₄)₂ x 12 H₂O, 3 mg L⁻¹ H₃BO₃, 0.3 µg L⁻¹ Na₂MoO₄ × 2 H₂O, 0.2 mg L⁻¹ Na₂SeO₃, 0.2 mg L⁻¹ NiCl₂ × 6 H₂O, 0.2 m,g L⁻¹ Na₂WO₄ × 6 H₂O, 20 µg L⁻¹ d-biotin, 20 µg L⁻¹ folic acid, 10 µg L⁻¹ pyridoxine-HCI, 50 µg L⁻¹ thiamine-HCI, 50 µg L⁻¹ riboflavin, 50 µg L⁻¹ nicotinic acid, 50 µg L⁻¹ Ca-pantothenate, 50 µg L⁻¹ vitamin B₁₂, 50 µg L⁻¹ p-aminobenzoate, 50 µg L⁻¹ lipoic acid, 10 mg L⁻¹ FeCl₃, 20 g L⁻¹ N-morpholinoethansulfonic acid (MES) with additional 500 mg L⁻¹ L-cysteine-hydrochloride. The pH at the beginning was set to pH = 6.0. The autotrophic cultivation was carried out in a 1L pressure-resistant glass bottle at 37 °C, an agitation rate of 150 min⁻¹ and a gassing rate of 1 L h⁻¹ with a synthesis gas consisting of 60% H₂, 20% CO and 20% CO₂ in an open water bath shaker Innova 3100 from New Brunswick. The gas was dispersed into the medium through a microbubble sparger with a pore size of 10 µm, which was mounted in the center of the reactors. The pH in the pre-cultivation was not controlled. After the pre-cultivation, an adequate amount of cell suspension was centrifuged (10 min, 4200 min⁻¹) in order to reach an OD₆₀₀ₙₘ of 0.1 in the main cultivation. The pellets were resuspended in fresh medium and transferred into the main cultivation vessels. The experiments were carried out in the same defined medium as described above, but without MES and with 4.17 g L⁻¹ sodium acetate instead. Acrylic acid was added to medium at varying concentrations from 25 to 200 mg L⁻¹ in the form of sodium acrylic acid prior to inoculation. The pH at the beginning was set to 6.0 and was later held constant at a pH of 5.5 by automatic addition of ammonium hydroxide (2.5 M). The autotrophic cultivations were carried out in 1L pressure-resistant glass bottles with a liquid volume of 250 mL at 37 °C and an agitation rate of 150 min⁻¹ in an open water bath shaker Innova 3100 from New Brunswick. The synthesis gas consisting of 60% H₂, 20% CO and 20% CO₂ was dispensed into the headspace of the reactor a gassing rate of 1 L h⁻¹. During the autotrophic cultivations, 5 mL samples were taken to determine OD₆₀₀ₙₘ, pH und product formation. The determination of the product concentrations was performed by semiquantitative 1H-NMR spectroscopy. As an internal quantification standard sodium trimethylsilylpropionate (T(M)SP) was used.

In the control cultivation without acrylate, cells began to grow until an OD₆₀₀ of 0.8 after 48 hours of cultivation. Acetate and ethanol concentrations reached concentrations of 6300 mg L⁻¹ and 2200 mg L⁻¹ respectively. Similar ethanol concentrations were reached in the other cultivations with added acrylate concentrations of up to 200 mg L⁻¹. With rising concentrations of acrylate, acetate concentrations reached lower values compared to the control cultivation. In all cultivations with added acrylate, the OD₆₀₀ reached after 48 hours was lower than in the control cultivation, what indicates a slight negative influence on growth and acetate formation with acrylate concentrations of up to 200 mg L-¹. Maximum allyl concentrations of 60 mg L⁻¹ were measured. Results shown in Table 2.

| **experiment 3.1 (control)** | | | NMR analytics | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,89 | 0,10 | 3380 | 21 | n.d. | n.d. |
| 3,9 | 5,84 | 0,14 | 3520 | 120 | n.d. | n.d. |
| 20,5 | 5,55 | 0,43 | 3998 | 926 | n.d. | n.d. |
| 28,0 | 5,49 | 0,55 | 4112 | 1418 | n.d. | n.d. |
| 44,4 | 5,41 | 0,75 | 5885 | 2168 | n.d. | n.d. |
| 47,7 | 5,39 | 0,81 | 6321 | 2261 | n.d. | n.d. |

| **experiment 3.2** | | | | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,89 | 0,10 | 3450 | 11 | 27 | n.d. |
| 3,9 | 5,85 | 0,14 | 3400 | 110 | 22 | n.d. |
| 20,5 | 5,55 | 0,43 | 3900 | 810 | 160 | n.q. |
| 28,0 | 5,49 | 0,50 | 4050 | 1150 | 150 | 16 |
| 44,4 | 5,42 | 0,58 | 4500 | 2100 | 110 | 36 |
| 47,7 | 5,42 | 0,57 | 4650 | 2300 | 110 | 36 |

| **experiment 3.3** | | | | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,89 | 0,10 | 3350 | 11 | 77 | n.d. |
| 3,9 | 5,84 | 0,14 | 3400 | 130 | 74 | n.d. |
| 20,5 | 5,60 | 0,41 | 3750 | 830 | 180 | n.d. |
| 28,0 | 5,49 | 0,48 | 4100 | 1250 | 160 | 17 |
| 44,4 | 5,42 | 0,56 | 4200 | 2150 | 110 | 47 |
| 47,7 | 5,44 | 0,55 | 4150 | 2300 | 110 | 50 |

**Table 2. Results of experiments 3.1-3.4 during batch autotrophic cultivation of Clostridium autoethanogenum on synthesis gas with carbon monoxide**

| **experiment 3.4** | | | | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,89 | 0,10 | 3500 | 21 | n.d. | n.d. |
| 3,9 | 5,86 | 0,14 | 3591 | 107 | n.d. | n.d. |
| 20,5 | 5,63 | 0,39 | 3850 | 920 | 210 | n.d. |
| 28,0 | 5,57 | 0,46 | 3900 | 1300 | 170 | 26 |
| 44,4 | 5,46 | 0,60 | 3800 | 2200 | 120 | 47 |
| 47,7 | 5,50 | 0,56 | 3800 | 2400 | 110 | 60 |

### Example 4

### Continuous conversion of acrylic acid to allyl alcohol during autotrophic cultivation of Clostridium autoethanogenum with continuous medium exchange

For a continuous conversion of acrylic acid to allyl alcohol the acetogenic bacterium *Clostridium autoethanogenum* DSM 10061 was cultivated on synthesis gas with 20% carbon monoxide with continuous medium exchange. All cultivation steps were carried out under anaerobic conditions in pressure-resistant glass bottles that were closed airtight with butyl rubber stoppers.

For the pre-culture 500 ml of a defined medium were inoculated with 5 mL of a frozen cryo stock of *C. autoethanogenum.* The defined medium was composed as follows: 0.5 g L⁻¹ MgCl₂ × 6 H₂O, 0.21 g L⁻¹ NaCl, 0.125 g L⁻¹ CaCl₂ x 2 H₂O, 2.65 g L⁻¹ NaH₂PO₄ × 2 H₂O, 0.5 g L⁻¹ KCI, 2.5 g L⁻¹ NH₄Cl, 15 mg L⁻¹ nitrilotriacetic acid, 30 mg L⁻¹ MgSO₄ x 7 H₂O, 5 mg L⁻¹ MnSO₄ x H₂O, 1 mg L⁻¹ FeSO₄ x 7 H₂O, 8 mg L⁻¹ Fe(SO₄)₂(NH₄)₂ x 6 H₂O, 2 mg L⁻¹ CoCl₂ x 6 H₂O, 2 mg L⁻¹ ZnSO₄ x 7 H₂O, 0.2 mg L⁻¹ CuCl₂ x 2 H₂O, 0.2 mg L⁻¹ KAI(SO₄)₂ x 12 H₂O, 3 mg L⁻¹ H₃BO₃, 0.3 µg L⁻¹ Na₂MoO₄ x 2 H₂O, 0.2 mg L⁻¹ Na₂SeO₃, 0.2 mg L⁻¹ NiCl₂ x 6 H₂O, 0.2 m,g L⁻¹ Na₂WO₄ x 6 H₂O, 20 µg L⁻¹ d-biotin, 20 µg L⁻¹ folic acid, 10 µg L⁻¹ pyridoxine-HCI, 50 µg L⁻¹ thiamine-HCI, 50 µg L⁻¹ riboflavin, 50 µg L⁻¹ nicotinic acid, 50 µg L⁻¹ Ca-pantothenate, 50 µg L⁻¹ vitamin B₁₂, 50 µg L⁻¹ p-aminobenzoate, 50 µg L⁻¹ lipoic acid, 10 mg L⁻¹ FeCl₃, 20 g L⁻¹ N-morpholinoethansulfonic acid (MES) with additional 500 mg L⁻¹ L-cysteine-hydrochloride. The pH at the beginning was set to pH = 6.0. The autotrophic cultivation was carried out in a 1L pressure-resistant glass bottle at 37 °C, an agitation rate of 150 min⁻¹ and a gassing rate of 1 L h⁻¹ with a synthesis gas consisting of 60% H₂, 20% CO and 20% CO₂ in an open water bath shaker Innova 3100 from New Brunswick. The gas was dispersed into the medium through a microbubble sparger with a pore size of 10 µm, which was mounted in the center of the reactors. The pH in the pre-cultivation was not controlled. After the pre-cultivation, an adequate amount of cell suspension was centrifuged (10 min, 4200 min⁻¹) in order to reach an OD₆₀₀ₙₘ of 0.1 in the main cultivation. The pellets were resuspended in fresh medium and transferred into the main cultivation vessel. The main experiment was carried out in the same defined medium as described above, but without MES and with 4.17 g L⁻¹ sodium acetate instead. The pH at the beginning was held constant at a pH of 5.5 by automatic addition of ammonium hydroxide (2.5 M). The autotrophic cultivation was carried out in a 500mL pressure-resistant glass bottle with a liquid volume of 400 mL at 37 °C and an agitation rate of 150 min⁻¹ in an open water bath shaker Innova 3100 from New Brunswick. Medium was continuously exchanged with a dilution rate of D ~ 1 d⁻¹. Cell mass was fully retained by microfiltration hollow fiber membranes made of polysulfone with a pore size of 0.45 µm. Membrane area was approximately 30 cm². Prior to inoculation, an acrylic acid concentration of 80 mg L⁻¹ was present in the medium. The fresh medium was mixed with acrylic acid in the form of sodium acrylic acid at rising concentrations, so that the acrylate concentration in the fermentation reactor steadily increased from 80 to 200 mg L-¹. The synthesis gas consisting of 60% H₂, 20% CO and 20% CO₂ was dispersed into the liquid phase by a hollow fiber membrane sparger with a pore size of 0.1 µm at a gassing rate of 3 L h⁻¹. During the autotrophic cultivations, 5 mL samples were taken to determine OD₆₀₀ₙₘ, pH und product formation. The determination of the product concentrations was performed by semiquantitative 1H-NMR spectroscopy. As an internal quantification standard sodium trimethylsilylpropionate (T(M)SP) was used.

During the autotrophic cultivation with continuous medium exchange, a constant conversion of acrylic acid to allyl alcohol was measured. Acetate and ethanol concentrations in the fermentation broth increased slowly until an acrylate concentration of approximately 100 mg L⁻¹ was reached and decreased thereafter. An increase in OD₆₀₀ could be measured until an acrylate concentration of 150 mg L⁻¹ was reached. A constant conversion of acrylic acid to allyl alcohol was measured with a maximum allyl alcohol concentration of 57 mg L⁻¹ reached after 184 hours. Results shown in Table 3.

**Table 3. Results of experiments 4 during autotrophic cultivation of Clostridium autoethanogenum with continuous medium exchange.**

| **experiment 4** | | | NMR analytics | | | |
|---|---|---|---|---|---|---|
| time, h | pH | OD 600 nm | acetate, mg/L | ethanol, mg/L | acrylate, mg/L | allyl alcohol, mg/L |
| 0,0 | 5,65 | 0,17 | 3350 | 76 | 84 | n.d. |
| 15,9 | 5,60 | 0,34 | 3700 | 340 | 95 | n.q. |
| 19,1 | 5,55 | 0,43 | 3500 | 380 | 91 | n.q. |
| 23,6 | 5,50 | 0,52 | 3650 | 500 | 91 | 11 |
| 40,6 | 5,41 | 0,82 | 3750 | 890 | 77 | 27 |
| 43,1 | 5,49 | 0,85 | 3700 | 890 | 73 | 21 |
| 47,0 | 5,49 | 0,89 | 3850 | 950 | 75 | 21 |
| 64,2 | 5,49 | 1,15 | 3900 | 1150 | 83 | 27 |
| 67,0 | 5,50 | 1,15 | 3950 | 1200 | 87 | 28 |
| 70,7 | 5,54 | 1,22 | 3750 | 1150 | 88 | 30 |
| 135,7 | 5,63 | 1,51 | 3450 | 1000 | 130 | 31 |
| 139,3 | 5,65 | 1,51 | 3350 | 1050 | 120 | 33 |
| 145,0 | 5,68 | 1,65 | 3450 | 1100 | 130 | 34 |
| 160,1 | 5,73 | 1,70 | 3300 | 1300 | 130 | 38 |
| 168,0 | 5,70 | 1,76 | 3250 | 1450 | 130 | 44 |
| 183,8 | 5,69 | 1,84 | 3250 | 1450 | 150 | 57 |
| 191,8 | 5,65 | 1,71 | 3400 | 1350 | 160 | 51 |
| 207,8 | 5,54 | 1,47 | 3450 | 860 | 180 | 37 |
| 214,8 | 5,54 | 1,42 | 3450 | 700 | 190 | 28 |
| 231,8 | 5,55 | 1,35 | 3150 | 470 | 190 | 15 |

### Example 5

### Conversion of acrylic acid to allyl alcohol during batch autotrophic cultivation of Clostridium ljungdahlii on synthesis gas with carbon monoxide

For a potential conversion of acrylic acid to allyl alcohol the acetogenic bacterium *Clostridium ljungdahlii* DSM 13528 is cultivated on synthesis gas with 25% carbon monoxide. All cultivation steps are carried out under anaerobic conditions in pressure-resistant glass bottles that are closed airtight with butyl rubber stoppers.

For the pre-culture 500 ml of a complex medium are inoculated with 5 mL of a frozen cryo stock of *C. ljungdahlii.* The medium was composed as follows: 1 g/L NH₄Cl, 0.1 g/L KCI, 0.2 g/L MgSO₄ x 7 H₂O, 0.8 g/L NaCl, 0.1 g/L KH₂PO₄, 20 mg/L CaCl₂ x 2 H₂O, 20 g/L MES, 1 g/L yeast extract, 0.4 g/L L-cysteine-HCI, 0.4 g/L Na₂S x 9 H₂O, 20 mg/L nitrilotriacetic acid, 10 mg/LMnSO₄ x H₂O, 8 mg/L (NH₄)₂Fe(SO₄)₂ x 6 H₂O, 2 mg/L CoCl₂ x 6 H₂O, 2 mg/L ZnSO₄ x 7 H₂O, 0.2 mg/L CuCl₂ x 2 H₂O, 0.2 mg/L Na₂MoO₄ x 2 H₂O, 0.2 mg/L NiCl₂ x 6 H₂O, 0.2 mg/L Na₂SeO₄, 0.2 mg/L Na₂WO₄ x 2 H₂O, 20 µg/L biotin, 20 µg/L folic acid, 100 µg/L pyridoxine-HCI, 50 µg/L thiamine-HCI x H₂O, 50 µg/L riboflavin, 50 µg/L nicotinic acid, 50 µg/L Ca-pantothenoic acid, 1 µg/L vitamine B12, 50 µg/L p-aminobenzoic acid, 50 µg/L lipoic acid and 20 g L⁻¹ N-morpholinoethansulfonic acid (MES). The pH at the beginning is set to pH = 6.0 and not controlled thereafter. The autotrophic cultivation is carried out in a 1L pressure-resistant glass bottle at 37 °C, an agitation rate of 150 min⁻¹ and a gassing rate of 1 L h⁻¹ with a synthesis gas consisting of 5% H₂, 25% CO, 25% CO₂ and 45% N₂ in an open water bath shaker Innova 3100 from New Brunswick. The gas is dispersed into the medium through a microbubble sparger with a pore size of 10 µm, which is mounted in the center of the reactors.

After the pre-cultivation, an adequate amount of cell suspension was centrifuged (10 min, 4200 min⁻¹) in order to reach an OD₆₀₀ₙₘ of 0.1 in the main cultivation. The pellets are resuspended in fresh medium and transferred into the main cultivation vessel. The experiments are carried out in the same medium as described above. Acrylic acid is added to medium at a concentration of 150 mg L⁻¹ in the form of sodium acrylic acid prior to inoculation. The pH at the beginning is set to 6.0 and is not controlled throughout the experiment. The autotrophic cultivations are carried out in 1L pressure-resistant glass bottles with a liquid volume of 500 mL at 37 °C and an agitation rate of 150 min⁻¹ in an open water bath shaker Innova 3100 from New Brunswick. The synthesis gas consisting of 5% H₂, 25% CO, 25% CO₂ and 45% N₂ is dispersed into the medium a gassing rate of 1 L h⁻¹ through a microbubble sparger with a pore size of 10 µm, which is mounted in the center of the reactors. During the autotrophic cultivations, 5 mL samples are taken to determine OD₆₀₀ₙₘ, pH und product formation. The determination of the product concentrations are performed by semiquantitative 1H-NMR spectroscopy. As an internal quantification standard sodium trimethylsilylpropionate (T(M)SP) is used.

In the control cultivation without acrylate, cells began to grow until an OD₆₀₀ of 1.74 after 159.5 hours of cultivation. Acetate and ethanol concentrations reached maximum concentrations of 7150 mg L⁻¹ and 680 mg L⁻¹ respectively.

In the cultivation with added acrylate, a similar OD₆₀₀, is reached and acrylate is reduced to allyl alcohol parallel to the formation of ethanol.

### experiment 5.1 (control)

**Table 4. Results of control experiment 5.1 during batch autotrophic cultivation of Clostridium ljungdahlii on synthesis gas with carbon monoxide and no acrylic acid.**

| time, h | pH | OD 600 nm | acetate, mg/L | ethanol, mg/L |
|---|---|---|---|---|
| 0.0 | 5.99 | 0.07 | 43 | 5 |
| 15.8 | 5.89 | 0.36 | 600 | 25 |
| 63.5 | 5.15 | 1.23 | 3100 | 47 |
| 136.0 | 4.52 | 1.66 | 6450 | 400 |
| 159.5 | 4.44 | 1.74 | 7150 | 540 |
| 183.5 | 4.47 | 1.74 | 7150 | 680 |

| | | | | |
|---|---|---|---|---|
| n.d. = not detectable | | | | |

### Example 6

### Conversion of acrylic acid to allyl alcohol during batch autotrophic cultivation of Clostridium ragsdalei on synthesis gas with carbon monoxide

For a potential conversion of acrylic acid to allyl alcohol the acetogenic bacterium *Clostridium ragsdalei* DSM 15248 is cultivated on synthesis gas with 25% carbon monoxide. All cultivation steps are carried out under anaerobic conditions in pressure-resistant glass bottles that were closed airtight with butyl rubber stoppers.

For the pre-culture 500 ml of a complex medium is inoculated with 5 mL of a frozen cryo stock of *C. ragsdalei.* The medium is composed as follows: 1 g/L NH₄Cl, 0.1 g/L KCI, 0.2 g/L MgSO₄ x 7 H₂O, 0.8 g/L NaCl, 0.1 g/L KH₂PO₄, 20 mg/L CaCl₂ x 2 H₂O, 20 g/L MES, 1 g/L yeast extract, 0.4 g/L L-cysteine-HCI, 0.4 g/L Na₂S x 9 H₂O, 20 mg/L nitrilotriacetic acid, 10 mg/LMnSO₄ x H₂O, 8 mg/L (NH₄)₂Fe(SO₄)₂ x 6 H₂O, 2 mg/L CoCl₂ x 6 H₂O, 2 mg/L ZnSO₄ x 7 H₂O, 0.2 mg/L CuCl₂ x 2 H₂O, 0.2 mg/L Na₂MoO₄ x 2 H₂O, 0.2 mg/L NiCl₂ x 6 H₂O, 0.2 mg/L Na₂SeO₄, 0.2 mg/L Na₂WO₄ x 2 H₂O, 20 µg/L biotin, 20 µg/L folic acid, 100 µg/L pyridoxine-HCI, 50 µg/L thiamine-HCI x H₂O, 50 µg/L riboflavin, 50 µg/L nicotinic acid, 50 µg/L Ca-pantothenoic acid, 1 µg/L vitamine B12, 50 µg/L p-aminobenzoic acid, 50 µg/L lipoic acid and 20 g L⁻¹ N-morpholinoethansulfonic acid (MES). The pH at the beginning is set to pH = 6.0 and not controlled thereafter. The autotrophic cultivation is carried out in a 1L pressure-resistant glass bottle at 37 °C, an agitation rate of 150 min⁻¹ and a gassing rate of 1 L h⁻¹ with a synthesis gas consisting of 5% H₂, 25% CO, 25% CO₂ and 45% N₂ in an open water bath shaker Innova 3100 from New Brunswick. The gas is dispersed into the medium through a microbubble sparger with a pore size of 10 µm, which is mounted in the center of the reactors.

After the pre-cultivation, an adequate amount of cell suspension is centrifuged (10 min, 4200 min⁻¹) in order to reach an OD₆₀₀ₙₘ of 0.1 in the main cultivation. The pellets are resuspended in fresh medium and transferred into the main cultivation vessel. The experiments are carried out in the same medium as described above. Acrylic acid is added to medium at a concentration of 150 mg L⁻¹ in the form of sodium acrylic acid prior to inoculation. The pH at the beginning is set to 6.0 and is not controlled throughout the experiment. The autotrophic cultivations are carried out in 1L pressure-resistant glass bottles with a liquid volume of 500 mL at 37 °C and an agitation rate of 150 min⁻¹ in an open water bath shaker Innova 3100 from New Brunswick. The synthesis gas consisting of 5% H₂, 25% CO, 25% CO₂ and 45% N₂ is dispersed into the medium a gassing rate of 1 L h⁻¹ through a microbubble sparger with a pore size of 10 µm, which is mounted in the center of the reactors. During the autotrophic cultivations, 5 mL samples are taken to determine OD₆₀₀ₙₘ, pH und product formation. The determination of the product concentrations is performed by semiquantitative 1H-NMR spectroscopy. As an internal quantification standard sodium trimethylsilylpropionate (T(M)SP) is used.

In the control cultivation without acrylate, cells began to grow until an OD₆₀₀ of 0.88 after 159.5 hours of cultivation. Acetate and ethanol concentrations reached maximum concentrations of 6250 mg L⁻¹ and 490 mg L⁻¹ respectively.

In the cultivation with added acrylate, a similar OD₆₀₀, is reached and acrylate is reduced to allyl alcohol parallel to the formation of ethanol.

### experiment 6.1 (control)

**Table 5. Results of control experiment 6.1 during batch autotrophic cultivation of Clostridium ragsdalei on synthesis gas with carbon monoxide and no acrylic acid.**

| time, h | pH | OD 600 nm | acetate, mg/L | ethanol, mg/L |
|---|---|---|---|---|
| 0.0 | 5.97 | 0.07 | 12 | 2 |
| 63.5 | 5.29 | 0.66 | 2900 | 17 |
| 136.0 | 4.72 | 0.86 | 5500 | 400 |
| 159.5 | 4.70 | 0.88 | 6250 | 490 |

| | | | | |
|---|---|---|---|---|
| n.d. = not detectable | | | | |

### Example 7

### Conversion of acrylic acid to allyl alcohol during batch autotrophic cultivation of Clostridium carboxidivorans on synthesis gas with carbon monoxide

For a potential conversion of acrylic acid to allyl alcohol the acetogenic bacterium *Clostridium carboxidivorans* DSM 15243 is cultivated on synthesis gas with 25% carbon monoxide. All cultivation steps are carried out under anaerobic conditions in pressure-resistant glass bottles that are closed airtight with butyl rubber stoppers.

For the pre-culture 500 ml of a complex medium is inoculated with 5 mL of a frozen cryo stock of *C. carboxidivorans.* The medium is composed as follows: 1 g/L NH₄Cl, 0.1 g/L KCI, 0.2 g/L MgSO₄ x 7 H₂O, 0.8 g/L NaCl, 0.1 g/L KH₂PO₄, 20 mg/L CaCl₂ x 2 H₂O, 20 g/L MES, 1 g/L yeast extract, 0.3 g/L L-cysteine-HCI, 20 mg/L nitrilotriacetic acid, 10 mg/LMnSO₄ x H₂O, 8 mg/L (NH₄)₂Fe(SO₄)₂ x 6 H₂O, 2 mg/L CoCl₂ x 6 H₂O, 2 mg/L ZnSO₄ x 7 H₂O, 0.2 mg/L CuCl₂ x 2 H₂O, 0.2 mg/L Na₂MoO₄ x 2 H₂O, 0.2 mg/L NiCl₂ x 6 H₂O, 0.2 mg/L Na₂SeO₄, 0.2 mg/L Na₂WO₄ x 2 H₂O, 20 µg/L biotin, 20 µg/L folic acid, 100 µg/L pyridoxine-HCI, 50 µg/L thiamine-HCI x H₂O, 50 µg/L riboflavin, 50 µg/L nicotinic acid, 50 µg/L Ca-pantothenoic acid, 1 µg/L vitamine B12, 50 µg/L p-aminobenzoic acid, 50 µg/L lipoic acid and 20 g L⁻¹ N-morpholinoethansulfonic acid (MES). The pH at the beginning is set to pH = 5.9 and not controlled thereafter. The autotrophic cultivation is carried out in a 1L pressure-resistant glass bottle at 37 °C, an agitation rate of 150 min⁻¹ and a gassing rate of 1 L h⁻¹ with a synthesis gas consisting of 5% H₂, 25% CO, 25% CO₂ and 45% N₂ in an open water bath shaker Innova 3100 from New Brunswick. The gas is dispersed into the medium through a microbubble sparger with a pore size of 10 µm, which is mounted in the center of the reactors.

After the pre-cultivation, an adequate amount of cell suspension is centrifuged (10 min, 4200 min⁻¹) in order to reach an OD₆₀₀ₙₘ of 0.1 in the main cultivation. The pellets are resuspended in fresh medium and transferred into the main cultivation vessel. The experiments are carried out in the same medium as described above. Acrylic acid is added to medium at a concentration of 150 mg L⁻¹ in the form of sodium acrylic acid prior to inoculation. The pH at the beginning is set to 5.9 and is not controlled throughout the experiment. The autotrophic cultivations are carried out in 1L pressure-resistant glass bottles with a liquid volume of 500 mL at 37 °C and an agitation rate of 150 min⁻¹ in an open water bath shaker Innova 3100 from New Brunswick. The synthesis gas consisting of 5% H₂, 25% CO, 25% CO₂ and 45% N₂ is dispersed into the medium a gassing rate of 1 L h⁻¹ through a microbubble sparger with a pore size of 10 µm, which is mounted in the center of the reactors. During the autotrophic cultivations, 5 mL samples were taken to determine OD₆₀₀ₙₘ, pH and product formation. The determination of the product concentrations is performed by semiquantitative 1H-NMR spectroscopy. As an internal quantification standard sodium trimethylsilylpropionate (T(M)SP) is used.

In the control cultivation without acrylate, cells began to grow until an OD₆₀₀ of 0.68 after 159.5 hours of cultivation. Acetate, ethanol, butyrate and butanol concentrations reached maximum concentrations of 2800 mg L⁻¹, 690 mg L-¹, 480 mg L⁻¹ and 230 mg L⁻¹ respectively.

In the cultivation with added acrylate, a similar OD₆₀₀, is reached and acrylate is reduced to allyl alcohol parallel to the formation of ethanol and butanol.

**Table 6. Results of control experiment 6.1 during batch autotrophic cultivation of Clostridium carboxidivorans on synthesis gas with carbon monoxide and no acrylic acid.**

| **experiment 7.1 (control)** | | | NMR analytics | | | |
|---|---|---|---|---|---|---|
| time, h | pH | OD 600 nm | acetate, mg/L | ethanol, mg/L | butyrate, mg/L | butanol, mg/L |
| 0.0 | 5.87 | 0.07 | 13 | 6 | n.d. | n.d. |
| 15.8 | 5.72 | 0.40 | 820 | 47 | n.d. | n.d. |
| 39.5 | 5.36 | 0.61 | 2100 | 72 | 16 | n.d. |
| 63.5 | 5.09 | 0.57 | 2800 | 150 | 120 | n.d. |
| 136.0 | 5.23 | 0.67 | 2000 | 610 | 430 | 190 |
| 159.5 | 5.25 | 0.68 | 2100 | 690 | 480 | 230 |

| | | | | | | |
|---|---|---|---|---|---|---|
| n.d. = not detectable | | | | | | |

### Example 8

### Conversion of acrylic acid to allyl alcohol during batch autotrophic cultivation of Alkalibaculum bacchi on synthesis gas with carbon monoxide

For a potential conversion of acrylic acid to allyl alcohol the acetogenic bacterium *Alkalibaculum bacchi* DSM 22112 is cultivated on synthesis gas with 25% carbon monoxide. All cultivation steps are carried out under anaerobic conditions in pressure-resistant glass bottles that are closed airtight with butyl rubber stoppers.

For the pre-culture 500 ml of a complex medium is inoculated with 5 mL of a frozen cryo stock of *A. bacchi.* The medium is composed as follows: 1 g/L NH₄Cl, 0.1 g/L KCI, 0.2 g/L MgSO₄ x 7 H₂O, 0.8 g/L NaCl, 0.1 g/L KH₂PO₄, 20 mg/L CaCl₂ x 2 H₂O, 20 g/L MES, 1 g/L yeast extract, 20 mg/L nitrilotriacetic acid, 10 mg/LMnSO₄ x H₂O, 8 mg/L (NH₄)₂Fe(SO₄)₂ x 6 H₂O, 2 mg/L CoCl₂ x 6 H₂O, 2 mg/L ZnSO₄ x 7 H₂O, 0.2 mg/L CuCl₂ x 2 H₂O, 0.2 mg/L Na₂MoO₄ x 2 H₂O, 0.2 mg/L NiCl₂ x 6 H₂O, 0.2 mg/L Na₂SeO₄, 0.2 mg/L Na₂WO₄ x 2 H₂O, 20 µg/L biotin, 20 µg/L folic acid, 100 µg/L pyridoxine-HCI, 50 µg/L thiamine-HCI x H₂O, 50 µg/L riboflavin, 50 µg/L nicotinic acid, 50 µg/L Ca-pantothenoic acid, 1 µg/L vitamine B12, 50 µg/L p-aminobenzoic acid, 50 µg/L lipoic acid and 20 g L⁻¹ N-morpholino-ethansulfonic acid (MES). The pH at the beginning is set to pH = 7.0 and not controlled thereafter. The autotrophic cultivation is carried out in a 1L pressure-resistant glass bottle at 37 °C, an agitation rate of 150 min⁻¹ and a gassing rate of 1 L h⁻¹ with a synthesis gas consisting of 5% H₂, 25% CO, 25% CO₂ and 45% N₂ in an open water bath shaker Innova 3100 from New Brunswick. The gas is dispersed into the medium through a microbubble sparger with a pore size of 10 µm, which is mounted in the center of the reactors.

After the pre-cultivation, an adequate amount of cell suspension is centrifuged (10 min, 4200 min⁻¹) in order to reach an OD₆₀₀ₙₘ of 0.1 in the main cultivation. The pellets are resuspended in fresh medium and transferred into the main cultivation vessel. The experiments are carried out in the same medium as described above. Acrylic acid is added to medium at a concentration of 150 mg L⁻¹ in the form of sodium acrylic acid prior to inoculation. The pH at the beginning is set to 7.0 and is not controlled throughout the experiment. The autotrophic cultivations are carried out in 1L pressure-resistant glass bottles with a liquid volume of 500 mL at 37 °C and an agitation rate of 150 min⁻¹ in an open water bath shaker Innova 3100 from New Brunswick. The synthesis gas consisting of 5% H₂, 25% CO, 25% CO₂ and 45% N₂ is dispersed into the medium a gassing rate of 1 L h⁻¹ through a microbubble sparger with a pore size of 10 µm, which is mounted in the center of the reactors. During the autotrophic cultivations, 5 mL samples are taken to determine OD₆₀₀ₙₘ, pH and product formation. The determination of the product concentrations was performed by semiquantitative 1H-NMR spectroscopy. As an internal quantification standard sodium trimethylsilylpropionate (T(M)SP) is used.

In the control cultivation without acrylate, cells began to grow until an OD₆₀₀ of 0.52 after 63.0 hours of cultivation. Acetate and ethanol concentrations reached maximum concentrations of 650 mg L⁻¹ and 770 mg L⁻¹ respectively.

In the cultivation with added acrylate, a similar OD₆₀₀, is reached and acrylate is reduced to allyl alcohol parallel to the formation of ethanol.

### experiment 8.1 (control)

**Table 7. Results of control experiment 6.1 during batch autotrophic cultivation of Alkalibaculum bacchi on synthesis gas with carbon monoxide and no acrylic acid.**

| time, h | pH | OD 600 nm | acetate, mg/L | ethanol, mg/L |
|---|---|---|---|---|
| 0.0 | 7.06 | 0.05 | 7 | 6 |
| 39.0 | 6.97 | 0.17 | 280 | 9 |
| 63.0 | 6.82 | 0.52 | 460 | 3 |
| 135.0 | 6.90 | 0.45 | 520 | 670 |
| 183.0 | 6.90 | 0.45 | 650 | 670 |
| 231.0 | 6.90 | 0.44 | 620 | 770 |

| | | | | |
|---|---|---|---|---|
| n.d. = not detectable | | | | |

## Claims

1. A method of producing allyl alcohol from acrylic acid, the method comprising:
contacting at least one acetogenic bacteria with an aqueous medium comprising acrylic acid and/or an acrylate and a gas mixture comprising carbon monoxide, wherein the acetogenic bacteria is selected from the group consisting of *Alkalibaculum bacchi* DSM 22112, *Clostridium aceticum* DSM 1496, *Clostridium autoethanogenum*DSM 10061, DSM 19630 and DSM 23693, *Clostridium carboxidivorans* DSM 15243, *Clostridium coskatii* ATCC no. PTA-10522, *Clostridium drakei* ATCC BA-623, *Clostridium formicoaceticum* DSM 92, *Clostridium glycolicum* DSM 1288, *Clostridium ljungdahlii* DSM 13528, *Clostridium ljungdahlii C-01* ATCC 55988, *Clostridium ljungdahlii ERI-2* ATCC 55380, *Clostridium ljungdahlii O-52* ATCC 55989, *Clostridium mayombei* DSM 6539, *Clostridium methoxybenzovorans* DSM 12182, *Clostridium ragsdalei* DSM 15248, *Clostridium scatologenes* DSM 757, and *Clostridium species* ATCC 29797.

2. The method according to claim 1, wherein the acetogenic bacteria is selected from the group consisting of *Clostridium ljungdahlii* DSM 13528, *Clostridium ljungdahlii C-01* ATCC 55988, *Clostridium ljungdahlii ERI-2* ATCC 55380, *Clostridium ljungdahlii O-52* ATCC 55989, *Clostridium autoethanogenum* DSM 10061, *Clostridium autoethanogenum* DSM 19630 and *Clostridium autoethanogenum* DSM 23693.

3. The method according to any one of the preceding claims, wherein the gas mixture comprises 1-50% by volume carbon monoxide gas.

4. The method according to any one of the preceding claims, wherein the gas mixture comprises 5-20% by volume carbon monoxide gas.

5. The method according to any one of the preceding claims, wherein the gas mixture comprises 10-20% by volume carbon monoxide gas.

6. The method according to any one of the preceding claims, wherein the gas mixture comprises hydrogen.

7. The method according to claim 6, wherein the gas mixture comprises 50-85% by volume hydrogen gas.

8. The method according to any one of the preceding claims, wherein the gas mixture comprises 60% by volume hydrogen gas.

9. The method according to any one of the preceding claims, wherein the gas mixture comprises carbon dioxide.

10. The method according to any one of the preceding claims, wherein the acrylic acid is from sodium acrylic acid.

11. The method according to any one of the preceding claims, wherein the pH of the aqueous medium is maintained at pH 5 to 6.

12. The method according to any one of the preceding claims, wherein the aqueous medium is continuously exchanged for new aqueous medium.

13. The method according to any one of the preceding claims, wherein the concentration of acrylic acid is maintained at 20-450mg/L in the aqueous medium.

14. The method according to any one of the preceding claims, wherein the concentration of acrylic acid is maintained at about 100mg/L in the aqueous medium.

## Patentansprüche

1. Verfahren zur Herstellung von Allylalkohol aus Acrylsäure, wobei das Verfahren Folgendes umfasst: Inkontaktbringen mindestens eines acetogenen Bakteriums mit einem wässrigen Medium, das Acrylsäure und/oder ein Acrylat umfasst, und einem Gasgemisch, das Kohlenmonoxid umfasst, wobei das acetogene Bakterium aus der Gruppe bestehend aus *Alkalibaculum bacchi* DSM 22112, *Clostridium aceticum* DSM 1496, *Clostridium autoethanogenum* DSM 10061, DSM 19630 und DSM 23693, *Clostridium carboxidivorans* DSM 15243, *Clostridium coskatii* ATCC-Nr. PTA-10522, *Clostridium drakei* ATCC BA-623, *Clostridium formicoaceticum* DSM 92, *Clostridium glycolicum* DSM 1288, *Clostridium ljungdahlii* DSM 13528, *Clostridium ljungdahlii C-01* ATCC 55988, *Clostridium ljungdahlii ERI-2* ATCC 55380, *Clostridium ljungdahlii 0-52* ATCC 55989, *Clostridium mayombei* DSM 6539, *Clostridium methoxybenzovorans* DSM 12182, *Clostridium ragsdalei* DSM 15248, *Clostridium scatologenes* DSM 757 und *Clostridium species* ATCC 29797 ausgewählt ist.

2. Verfahren nach Anspruch 1, wobei das acetogene Bakterium aus der Gruppe bestehend aus *Clostridium ljungdahlii* DSM 13528, *Clostridium ljungdahlii C-01* ATCC 55988, *Clostridium ljungdahlii ERI-2* ATCC 55380, *Clostridium ljungdahlii O-52* ATCC 55989, *Clostridium autoethanogenum* DSM 10061, *Clostridium autoethanogenum* DSM 19630 und *Clostridium autoethanogenum* DSM 23693 ausgewählt ist.

3. Verfahren nach einem der vorhergehenden Ansprüche, wobei das Gasgemisch 1-50 Vol.-% Kohlenmonoxidgas umfasst.

4. Verfahren nach einem der vorhergehenden Ansprüche, wobei das Gasgemisch 5-20 Vol.-% Kohlenmonoxidgas umfasst.

5. Verfahren nach einem der vorhergehenden Ansprüche, wobei das Gasgemisch 10-20 Vol.-% Kohlenmonoxidgas umfasst.

6. Verfahren nach einem der vorhergehenden Ansprüche, wobei das Gasgemisch Wasserstoff umfasst.

7. Verfahren nach Anspruch 6, wobei das Gasgemisch 50 bis 85 Vol.-% Wasserstoffgas umfasst.

8. Verfahren nach einem der vorhergehenden Ansprüche, wobei das Gasgemisch 60 Vol.-% Wasserstoffgas umfasst.

9. Verfahren nach einem der vorhergehenden Ansprüche, wobei das Gasgemisch Kohlendioxid umfasst.

10. Verfahren nach einem der vorhergehenden Ansprüche, wobei die Acrylsäure aus Natriumacrylsäure stammt.

11. Verfahren nach einem der vorhergehenden Ansprüche, wobei der pH-Wert des wässrigen Mediums bei pH 5 bis 6 gehalten wird.

12. Verfahren nach einem der vorhergehenden Ansprüche, wobei das wässrige Medium kontinuierlich gegen neues wässriges Medium ausgetauscht wird.

13. Verfahren nach einem der vorhergehenden Ansprüche, wobei die Konzentration von Acrylsäure in dem wässrigen Medium bei 20-450 mg/l gehalten wird.

14. Verfahren nach einem der vorhergehenden Ansprüche, wobei die Konzentration von Acrylsäure in dem wässrigen Medium bei etwa 100 mg/l gehalten wird.

## Revendications

1. Procédé de production d'alcool allylique à partir d'acide acrylique, le procédé comprenant :
la mise en contact d'au moins une bactérie acétogène avec un milieu aqueux comprenant de l'acide acrylique et/ou un acrylate et un mélange de gaz comprenant du monoxyde de carbone, la bactérie acétogène étant choisie dans le groupe constitué par *Alkalibaculum bacchi* DSM 22112, *Clostridium aceticum* DSM 1496, *Clostridium autoethanogenum* DSM 10061, DSM 19630 et DSM 23693, *Clostridium carboxidivorans* DSM 15243, *Clostridium coskatii* n° ATCC PTA-10522, *Clostridium drakei* ATCC BA-623, *Clostridium formicoaceticum* DSM 92, *Clostridium glycolicum* DSM 1288, *Clostridium ljungdahlii* DSM 13528, *Clostridium ljungdahlii* C-01 ATCC 55988, *Clostridium ljungdahlii* ERI-2 ATCC 55380, *Clostridium ljungdahlii* 0-52 ATCC 55989, *Clostridium mayombei* DSM 6539, *Clostridium methoxybenzovorans* DSM 12182, *Clostridium ragsdalei* DSM 15248, *Clostridium scatologenes* DSM 757, et *Clostridium species* ATCC 29797.

2. Procédé selon la revendication 1, la bactérie acétogène étant choisie dans le groupe constitué par *Clostridium ljungdahlii* DSM 13528, *Clostridium ljungdahlii* C-01 ATCC 55988, *Clostridium ljungdahlii* ERI-2 ATCC 55380, *Clostridium ljungdahlii* 0-52 ATCC 55989, *Clostridium autoethanogenum* DSM 10061, *Clostridium autoethanogenum* DSM 19630 et *Clostridium autoethanogenum* DSM 23693.

3. Procédé selon l'une quelconque des revendications précédentes, le mélange de gaz comprenant de 1 à 50 % en volume de gaz de monoxyde de carbone.

4. Procédé selon l'une quelconque des revendications précédentes, le mélange de gaz comprenant de 5 à 20 % en volume de gaz de monoxyde de carbone.

5. Procédé selon l'une quelconque des revendications précédentes, le mélange de gaz comprenant de 10 à 20 % en volume de gaz de monoxyde de carbone.

6. Procédé selon l'une quelconque des revendications précédentes, le mélange de gaz comprenant de l'hydrogène.

7. Procédé selon la revendication 6, le mélange de gaz comprenant 50 à 85 % en volume de gaz d'hydrogène.

8. Procédé selon l'une quelconque des revendications précédentes, le mélange de gaz comprenant 60 % en volume de gaz d'hydrogène.

9. Procédé selon l'une quelconque des revendications précédentes, le mélange de gaz comprenant du dioxyde de carbone.

10. Procédé selon l'une quelconque des revendications précédentes, l'acide acrylique provenant du sel de sodium de l'acide acrylique.

11. Procédé selon l'une quelconque des revendications précédentes, le pH du milieu aqueux étant maintenu à pH 5 à 6.

12. Procédé selon l'une quelconque des revendications précédentes, le milieu aqueux étant échangé de manière continue avec un nouveau milieu aqueux.

13. Procédé selon l'une quelconque des revendications précédentes, la concentration d'acide acrylique étant maintenue à 20 à 450 mg/L dans le milieu aqueux.

14. Procédé selon l'une quelconque des revendications précédentes, la concentration d'acide acrylique étant maintenue à environ 100 mg/L dans le milieu aqueux.
